# EUROPÄISCHE PATENTSCHRIFT

(11) **EP 1 088 522 B1**
(45) Veröffentlichungstag und Bekanntmachung des Hinweises auf die Patenterteilung: **24.05.2006**
(21) Anmeldenummer: 00810782.3
(22) Anmeldetag: 31.08.2000
(51) Int. Cl.: A61B 17/80

(54) **Knochenplatte zum Schienen einer Bruchstelle an einem Knochen mit mehreren Knochenschrauben**
Bone plate with multiple bone screws for setting a fracture
Plaque d'ostéosynthèse avec plusieurs vis à os pour fixer une fracture

(30) Priorität: 30.09.1999 EP 99810881
(43) Veröffentlichungstag der Anmeldung: 04.04.2001
(73) Patentinhaber: Zimmer GmbH, 8404 Winterthur (CH)
(72) Erfinder: Bühler, Daniel W. Dr., 8304 Wallisellen (CH); Uhthoff, Hans K. Dr., Ottawa, Ontario K1M 1J2 (CA); Backman, David S., Gloucester, Ontario K1J 6L2 (CA)
(74) Vertreter: Manitz, Finsterwald & Partner GbR

(56) Entgegenhaltungen:
- EP-A- 0 052 998
- EP-A- 0 266 146
- GB-A- 2 305 483
- US-A- 4 943 292

## Beschreibung

Die Erfindung handelt von einer Knochenplatte zum Schienen einer Bruchstelle an einem Knochen mit mehreren Knochenschrauben, welche die Knochenplatte mit ihrem Schraubenkopf zum Knochen fixieren und welche in der Richtung zur Bruchstelle radial über ein elastisches Kunststoffpolster zur Knochenplatte abgestützt sind, um eine beschränkte Verschiebung bei Kompressionsbelastung des Knochens zuzulassen.

Knochenplatten üben im Gegensatz zu Druckplatten keine Kompression bei ihrem Anbringen auf die Bruchstelle eines Knochens aus. Während der Befestigung mit Knochenschrauben bleiben die Knochenteile unverändert in Längsrichtung zur Knochenplatte.

In der Patentschrift U.S. 4,943,292 (FOUX) (Basis für den Oberbegriff des Anspruchs 1) ist eine Knochenplatte mit Langlöchern gezeigt. Zwischen dem Befestigungskopf einer passenden Knochenschraube und der Knochenplatte ist eine Ringscheibe angebracht, welche mit einem Vorsprung in Form eines Polsters auf der Seite zu einer vorgesehenen Knochenbruchstelle in ein Langloch hineinragt. Als Werkstoff für Ringscheibe und Polster wird ein elastischer Kunststoff vorgesehen, der auch ein Gleiten des Schraubenkopfes ermöglichen soll, wenn das Polster bei einer Kompression des Knochens zusammengedrückt wird. Ein Problem dieser Anordnung besteht darin, dass die Knochenplatte, welche durch die Knochenschraube auf dem Knochen angepresst ist, relativ zum Knochen sich bewegen sollte. Das Anzugsmoment der Knochenschraube bestimmt zusammen mit der Reibung zwischen Platte und Knochen, die Grösse einer Kompressionskraft, die zu einer Verstellung in axialer Richtung führt. Da die Haftreibung praktisch immer grösser als die Gleitreibung ist, wird die Verstellung eher sprungartig erfolgen und ebenso eine Rückstellung durch die Polster, wenn die Kompressionskraft nachlässt. Ein weiterer Nachteil besteht darin, dass wegen der grossen Auflageflächen grosse Knochenanteile in ihrer Durchblutung gestört werden und in ihrer Knochendichte abnehmen (Porose).

Aufgabe der Erfindung ist es, diese Umstände zu verbessern. Sie löst die Aufgabe gemäss den Kennzeichen des unabhängigen Anspruchs 1, indem der Schraubenkopf die Knochenplatte vollständig durchdringt und mit einer Auflagefläche auf dem Knochen aufliegt, die mindestens um einen Betrag ε₁ über die Unterseite der Knochenplatte vorsteht und dass an das Kunststoffpolster ein Ringkörper angeformt ist, der mit dem Kunststoffpolster an der Unterseite der Knochenplatte ansetzbar ist und um einen Betrag ε₂ über die Unterseite vorsteht.

Die Erfindung hat den Vorteil, dass im Gegensatz zu Druckplatten, welche eine direkte, kallusfreie primäre Heilung anstreben, eine sekundäre Knochenheilung mit kontrollierter Kallusbildung ermöglicht wird. Durch die gezielte Verteilung der in der Achsrichtung der Knochenschraube wirksamen Anpresskraft auf eine am Knochen aufliegende Auflagefläche des Schraubenkopfes und auf einen Ringkörper als Zwischenglied sind die Reibungskräfte, die einer Längsverstellung der Knochenplatte entgegenwirken, so gering, dass die Federwirkung des Kunststoffpolsters wirklich zum Tragen kommt. Im Frakturspalt, der zur Kallusbildung führt, wird durch die gezielt zugelassenen Mikrobewegungen in der Längsrichtung der Knochenplatte die Kallusbildung angeregt und letztlich eine schnellere und solidere Heilung erreicht.

Der Schraubenkopf ist unabhängig von den Reibungskräften, die der Längsverstellung der Knochenplatte entgegenwirken, mit dem Knochen verspannt, während die Vorspannung auf die zwischen Schraubenkopf und Ringkörper geklemmte Knochenplatte klein gehalten werden kann. Da die Knochenplatte selbst hohl liegt und den Knochen kaum berührt, treten keine zusätzlichen Kräfte auf, die die Reibung bei den Mikrobewegungen beeinflussen.

Weitere Verbesserungen werden mit den Merkmalen der abhängigen Ansprüche 2 bis 12 erreicht. So ist es sinnvoll Ringkörper und Kunststoffpolster einstückig herzustellen und für die elastische Wirkung einen Kompressions-E-Modul E_{c} zwischen 500 und 3 000 MPa zu wählen. Durch die Wahl eines bioresorbierbaren Kunststoffes für Kunststoffpolster und Ring ergibt sich eine dem Heilungsprozess angepasste Langzeitwirkung. Dadurch, dass sich das bioresorbierbare Material im Verlauf von mehreren Wochen langsam abbaut, wird die Knochenplatte im Verlauf der Zeit immer weniger hart eingespannt und wird dem heilenden Knochen im Rahmen enger Grenzen eine stetig wachsende Belastung zugeteilt. Beispiele für ein solches Material sind Polylactide. Die Firma Boehringer, Ingelheim, Deutschland stellt unter der Produktebezeichnung Resomer R208 ein solches Polylactid her, welches sich durch Hydrolyse in etwa 30 Wochen im menschlichen Körper abbaut.

Ein weiterer Vorteil liegt darin, dass grosse Flächenpressungen auf kleine, vorgegebene Bereiche am Knochen beschränkt sind, wobei sich der Anteil vom Ringkörper mit dem Abbau des bioresorbierbaren Materials stetig verkleinert, sodass der Zeitpunkt für die Entfernung der Knochenplatte ohne grosse Schäden weiter hinausgezögert werden kann. Die Auflagefläche von Ringkörper und Schraubenkopf auf dem Knochen ist mehr als 100 % grösser als der Querschnitt des Nenndurchmessers des Gewindes der Knochenschraube. Es genügt, diese Auflagefläche kleiner als das achtfache vom Querschnitt des Nenndurchmessers des Gewindes zu halten, um innerhalb dieser Grenzen zwischen 100 % und 800 % eine Auflagefläche zu finden, die dem Gewinde der Knochenschraube bezüglich erzielbarer Auflagekraft angepasst ist.

Dadurch, dass die Ringkörper jeweils nur in einer Position in die Knochenplatte einsetzbar sind und in dieser Position einrasten, können die Kunststoffpolster, wenn sie mit dem Ringkörper verbunden sind, nur auf der Seite zur Bruchstelle hin montiert werden. Zur besseren Angleichung an die Rundung von Röhrenknochen, können Knochenplatte und Ringkörper auf ihrer Unterseite in Querrichtung konkav gewölbt sein.

Im folgenden wird die Erfindung anhand von Ausführungsbeispielen beschrieben. Es zeigen:
- Fig. 1: Schematisch einen Ausschnitt einer erfindungsgemässen Knochenplatte, die in der Nähe der Bruchstelle eines Knochens mit einer Knochenschraube fixiert ist;
- Fig. 2: schematisch eine Draufsicht auf einen Ringkörper mit einem sichelförmigen Kunststoffpolster;
- Fig. 3: schematisch einen Querschnitt durch den Ringkörper von Fig. 2;
- Fig. 4: schematisch einen Längsschnitt durch den Ringkörper von Fig. 3; und
- Fig. 5: schematisch einen Längsschnitt durch eine Knochenplatte mit jeweils drei fluchtenden Langlöchern beidseits der Bruchstelle eines Knochens.

Die Figuren zeigen eine Knochenplatte zum Schienen einer Bruchstelle 10 an einem Knochen 12 mit mehreren Knochenschrauben 2. Zwischen Knochenschrauben 2 und Knochenplatte 1 ist in der Richtung zur Bruchstelle 10 hin ein elastisches Kunststoffpolster 8 angebracht. Der Schraubenkopf 2a durchdringt die Knochenplatte 1 vollständig und liegt mit einer Auflagefläche 15 auf dem Knochen unter Vorspannung auf. An das Kunststoffpolster 8 ist ein Ringkörper 11 angeformt, der mit dem Kunststoffpolster 8 an der Unterseite 6 der Knochenplatte montierbar ist. Ringkörper 11 und Auflagefläche 15 des Schraubenkopfes 2a stehen um einen Betrag ε der nicht gleich gross sein muss über die Unterseite der Knochenplatte vor.

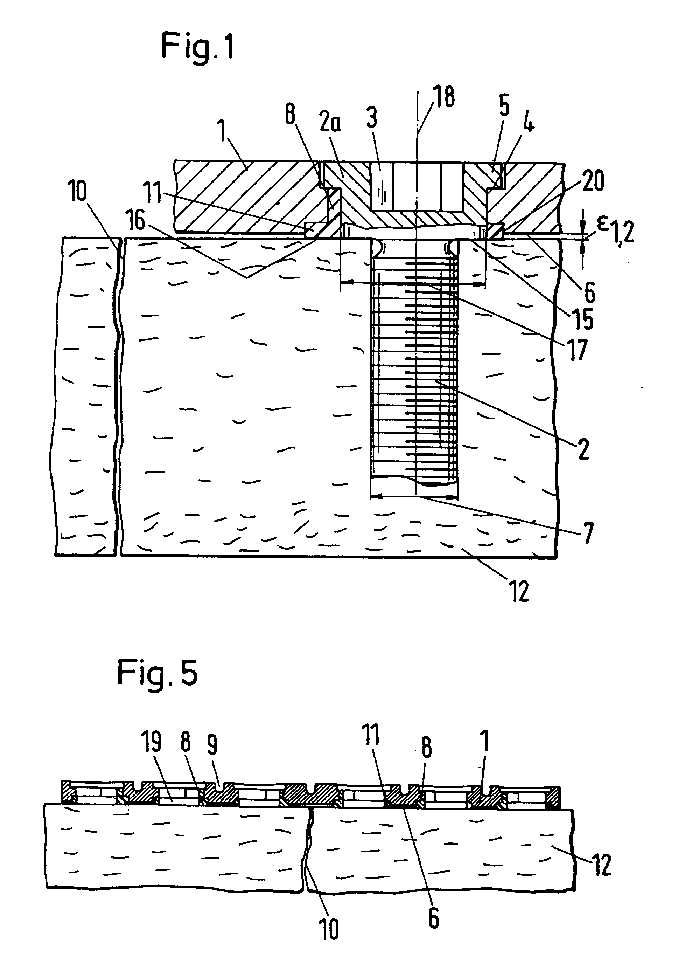

Ein Ausführungsbeispiel ist in den Figuren 1 bis 5 gezeigt. In allen Figuren sind die gleichen Hinweiszeichen verwendet. In Fig. 1 ist eine Knochenschraube 2 mit ihrem Gewinde mit einem Neundurchmesser 7 von 3,5 mm in einem Knochen 12 verankert und ist mit einer Auflagefläche 15 ihres Schraubenkopfes 2a, die einen Durchmesser 17 von 5,5 mm aufweist, gegen den Knochen 12 verspannt. Versuche haben gezeigt, dass der Knochen unter dieser Auflagefläche 15 bis zu 200 µm einsinken kann. Der vollständig in der Knochenplatte 1 versenkte Schraubenkopf 2a mit Innensechskant 3 besitzt im oberen Bereich eine Schulter 5, mit der er auf einem ringförmigen Absatz 4 in einer Bohrung 19 der Knochenplatte 1 aufliegt und deren Bewegung nach oben in der Richtung der Schraubenachse 18 begrenzt. Auf der Unterseite der Knochenplatte 1 ist ein Ringkörper 11 eingepresst und durch eine Schnappverbindung 20 gehalten. Die Auflagefläche 15 steht gegenüber der Unterseite 6 der Knochenplatte um einen Betrag ε₁ vor, der mindestens so gross ist, dass die Unterseite 6 nicht auf dem Knochen 12 aufliegt. Der Betrag ε₁ ist jeweils grösser als 0,2 mm gewählt, damit die Unterseite 6 der Knochenplatte zwischen den Knochenschrauben 2 mit Sicherheit hohl liegt. Der Ringkörper 11 steht ebenfalls mit seiner Auflagefläche 16 um einen ε₂, der kleiner sein kann als derjenige der Schraubenauflagefläche 15, gegenüber der Unterseite 6 vor, damit die Knochenplatte 1 nur mit einer begrenzten Kraft zwischen Ringkörper 11 und Schulter 5 verspannt ist. Eine Kompression des Knochens und eine Rückstellung ist soweit möglich, wie es das elastische an den Ringkörper 11 angeformte Kunststoffpolster 8 und die durch die Vorspannung erzeugte Reibung zwischen Schulter 5 und Absatz 4 zulassen. In Fig. 5 sind beidseitig der Bruchstelle 10 drei hintereinander liegende Bohrungen 19 angeordnet mit Ringkörpern 11 deren Kunststoffpolster 8 jeweils zur Bruchstelle 10 hin angeordnet sind. Die Bohrungen 19 sind als Langlöcher ausgeführt, damit neben dem einzusetzenden kreisförmigen Schraubenkopf 2a auch noch die Polster 8 Platz haben. Zwischen den Bohrungen 19 liegt die Unterseite der Knochenplatte hohl, während sie an der Oberseite Aussparungen 9 aufweist, welche die Anpassung an die Knochenform in Längsrichtung erleichtern. Ebenso sind zwischen den Bohrungen auf den Seiten der Knochenplatte Aussparungen zur Reduktion des Widerstandsmomentes möglich. In Querrichtung sind die Unterseite 6 und der Ringkörper 11 konkav gewölbt, um sich in Querrichtung der Knochenform anzupassen. Dadurch, dass das Material für Kunststoffpolster und Ringkörper bioresorbierbar ist, lassen sich die Auslenkungen der Mikrobewegungen zeitlich so steuern, dass mit dem Rücksetzen der Oberflächen von Polster und Ringkörper immer grössere aber noch verträgliche Druckspitzen an der Bruchstelle zugelassen werden. Der Knochen kann somit entsprechend dem Heilungsprozess seine tragende Funktion übernehmen, was sich auf die Knochenneubildung sehr positiv auswirkt.

Als bioresorbierbarer Kunststoff ist ein Poly-D, L-lactid vorgesehen, das sich durch Hydrolyse in etwa 30 Wochen abbaut; beispielsweise das Material Resomer R208 der Firma Boehringer, Ingelheim, Deutschland.

Das Material der Knochenplatte besteht aus Titan oder aus einer Titanlegierung wie Protasul^{™} der Anmelderin Sulzer Orthopedics Ltd..

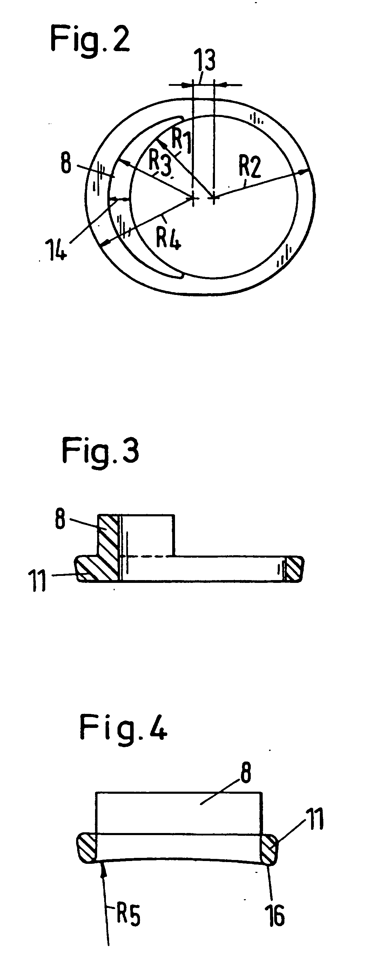

Die Figuren 2, 3 und 4 zeigen für ein solches bioresorbierbares Material einen als Spritzling hergestellten Ringkörper 11, an den ein sichelförmiges Polster 8 angeformt ist. Der Spritzling besitzt eine zylindrische Innenfläche mit Radius R₁ an welcher der Schraubenkopf mit seinem Durchmesser 17 anliegt. Die äussere Kontur der Sichelform wird durch einen Radius R₃ begrenzt dessen Radiusmittelpunkt um einen Versatz 13 in der Längsrichtung verschoben ist. Die Aussenkontur vom eigentlichen Ringkörper entsteht durch einen Radius R₂ mit gleichem Radiusmittelpunkt wie der Radius R₁ und durch einen Radius R₄ mit annähernd gleichem Radiusmittelpunkt wie Radius R₃, sowie durch die Tangenten an die Kreise R₂ und R₄. Das sichelförmige Polster 8 hat eine grösste Dicke 14, die 15 % vom Durchmesser 17 des anliegenden Schraubenkopfes 2a entspricht. Für einen grossen E-Modul kann dieser Wert auch kleiner gewählt werden. Allerdings sind dann bei gleicher Kompressionskraft die Mikrobewegungen geringer.

In Fig. 4 ist die Auflagefläche 16 des Ringkörpers 11 in Querrichtung konkav gewölbt mit einer Krümmung entsprechend einem Krümmungsradius R₅.

## Patentansprüche

1. Knochenplatte zum Schienen einer Bruchstelle (10) an einem Knochen (12) mit mehreren Knochenschrauben (2), welche die Knochenplatte (1) mit ihrem Schraubenkopf zum Knochen fixieren können und mit elastischen Kunststoffpolstern (8), die in Gebrauch die Knochenschrauben (2) in der Richtung zur Bruchstelle (10) radial abstützen, um eine beschränkte Verschiebung bei Kompressionsbelastung des Knochens zuzulassen, **dadurch gekennzeichnet, dass** in Gebrauch jeder Schraubenkopf (2a) die Knochenplatte vollständig durchdringt und mit einer Auflagefläche (15) auf dem Knochen aufliegt, die mindestens um einen Betrag ε₁ über die Unterseite (6) der Knochenplatte (1) vorsteht und dass an jedes Kunststoffpolster (8) ein Ringkörper (11) angeformt ist, der in Gebrauch mit dem Kunststoffpolster (8) an der Unterseite (6) der Knochenplatte ansetzbar ist und um einen Betrag ε₂ über die Unterseite (6) vorsteht.

2. Knochenplatte nach Anspruch 1, **dadurch gekennzeichnet, dass** das Material von Kunststoffpolster (8) und Ringkörper (11) einen Kompressions-E-Modul E_{c} zwischen 500 und 3 000 MPa aufweist.

3. Knochenplatte nach einem der Ansprüche 1 oder 2, **dadurch gekennzeichnet, dass** das Material von Kunststoffpolster (8) und Ringkörper (11) ein bioresorbierbares Material ist, welches sich erst nach mehreren Wochen abbaut.

4. Knochenplatte nach Anspruch 3, **dadurch gekennzeichnet, dass** das Material vom Kunststoffpolster (8) und Ringkörper (11) ein Polylactide ist, welches sich in ca. 30 Wochen abbaut.

5. Knochenplatte nach einem der Ansprüche 1 bis 4, **dadurch gekennzeichnet, dass** das Kunststoffpolster (8) in Sichelform an dem Schraubenkopf (2a) anliegt.

6. Knochenplatte nach Anspruch 5, **dadurch gekennzeichnet, dass** die Sichelform eine grösste Sicheldicke (14) in der Richtung der Längsachse der Knochenplatte (1) aufweist und dass die grösste Sicheldicke (14) mindestens 8 % vom Durchmesser (17) des anliegenden Schraubenkopfes (2a) entspricht.

7. Knochenplatte nach einem der Ansprüche 1 bis 6, **dadurch gekennzeichnet, dass** die über die Unterseite (6) vorstehenden Auflageflächen (15, 16) von Schraubenkopf (2a) und Ringkörper (11) mehr als 100 % grösser als der Querschnitt des Neundurchmessers (7) des Gewindes der Knochenschraube (2) ist.

8. Knochenplatte nach einem der Ansprüche 1 bis 7, **dadurch gekennzeichnet, dass** die Ringkörper (11) jeweils nur in einer Position in einer Bohrung (19) der Knochenplatte (1) einsetzbar sind und eine Schnappverbindung (20) zur Knochenplatte (1) aufweisen.

9. Knochenplatte nach einem der Ansprüche 1 bis 8, **dadurch gekennzeichnet, dass** Knochenplatte (1) und Ringkörper (11) auf ihrer Unterseite (6, 16) in Querrichtung konkav gewölbt sind.

10. Knochenplatte nach einem der Ansprüche 1 bis 9, **dadurch gekennzeichnet, dass** die Köpfe (2a) der Knochenschrauben (2) vollständig in der Knochenplatte (1) versenkt sind.

11. Knochenplatte nach einem der Ansprüche 1 bis 10, **dadurch gekennzeichnet, dass** der Querschnitt der Knochenplatte (1) durch Ausnehmungen (9) im Bereich zwischen den Bohrungen (19) geschwächt ist, um dort Bereiche mit geringerer Biegesteifigkeit zu schaffen.

12. Knochenplatte nach einem der Ansprüche 1 bis 11, **dadurch gekennzeichnet, dass** sie aus Titan oder einer Titanlegierung besteht.

## Claims

1. Bone plate for the splinting of a fracture (10) at a bone (12), with a plurality of bone screws (2) which can fix the bone plate (1) to the bone with their screw head and with elastic cushions of plastic (8) which radially support the bone screws (2) in use in the direction towards the fracture (10) in order to permit a restricted displacement on compression loading of the bone, **characterized in that** in use each screw head (2a) penetrates through the bone plate completely and lies in contact on the bone with a contact surface (15) which projects beyond the lower side (6) of the bone plate (1) by at least an amount ε₁; and **in that** a ring body (11) is molded to each cushion of plastic (8), can be placed in use with the cushion of plastic (8) at the lower side (6) of the bone plate and projects beyond the lower side (6) by an amount ε₂.

2. Bone plate in accordance with claim 1, **characterized in that** the material of the cushion of plastic (8) and of the ring body (11) has a compression E-module E_{c} between 500 and 3,000 MPa.

3. Bone plate in accordance with any one of the claims 1 or 2, **characterized in that** the material of the cushion of plastic (8) and of the ring body (11) is a bio-absorbable material which is decomposed only after a plurality of weeks.

4. Bone plate in accordance with claim 3, **characterized in that** the material of the cushion of plastic (8) and of the ring body (11) is a polyactide which is decomposed in approximately 30 weeks.

5. Bone plate in accordance with any one of the claims 1 to 4, **characterized in that** the cushion of plastic (8) contacts the screw head (2a) in the shape of a sickle.

6. Bone plate in accordance with claim 5, **characterized in that** the sickle shape has a maximum sickle thickness (14) in the direction of the longitudinal axis of the bone plate (1); and **in that** the maximum sickle thickness (14) corresponds to at least 8 % of the diameter (17) of the contacting screw head (2a).

7. Bone plate in accordance with any one of the claims 1 to 6, **characterized in that** the contact surfaces (15, 16) of the screw head (2a) and of the ring body (11) which project beyond the lower side (6) are more than 100 % larger than the cross-section of the nominal diameter (7) of the thread of the bone screw (2).

8. Bone plate in accordance with any one of the claims 1 to 7, **characterized in that** in each case the ring bodies (11) can be inserted in only one position in a bore (19) of the bone plate (1) and have a snap-in connection (20) to the bone plate (1).

9. Bone plate in accordance with any one of the claims 1 to 8, **characterized in that** the bone plate (1) and the ring body (11) are concavely arched on their lower side (6, 16) in the transverse direction.

10. Bone plate in accordance with any one of the claims 1 to 9, **characterized in that** the heads (2a) of the bone screws (2) are completely sunk in the bone plate (1).

11. Bone plate in accordance with any one of the claims 1 to 10, **characterized in that** cross-section of the bone plate (1) is weakened by cut-outs (9) in the region between the bores (19) in order to create regions with lower bending stiffness there.

12. Bone plate in accordance with any one of the claims 1 to 11, **characterized in that** it consists of titanium or of a titanium alloy.

## Revendications

1. Plaque d'ostéosynthèse pour mettre une attelle à une fracture (10) sur un os (12), comportant plusieurs vis à os (2) qui peuvent fixer la plaque d'ostéosynthèse (1) avec leur tête de vis vers l'os et des rembourrages élastiques en matière plastique (8) qui, à l'utilisation, soutiennent radialement les vis à os (2) dans la direction de la fracture (10) pour permettre un déplacement limité en cas de sollicitation de l'os par compression, **caractérisée en ce que** à l'utilisation, chaque tête de vis (2a) traverse totalement la plaque d'ostéosynthèse et est en appui sur l'os avec une surface d'appui (15) qui fait saillie d'au moins une valeur ε₁ au-dessus de la face inférieure (6) de la plaque d'ostéosynthèse et **en ce que** sur chaque rembourrage en matière plastique (8) est conformé un corps annulaire (11) qui, à l'utilisation, peut être rapporté avec le rembourrage en matière plastique (8) sur la face inférieure (6) de la plaque d'ostéosynthèse et qui fait saillie d'une valeur ε₂ au-dessus de la face inférieure (6).

2. Plaque d'ostéosynthèse selon la revendication 1, **caractérisée en ce que** le matériau du rembourrages en matière plastique (8) et du corps annulaire (11) présente un module de compression E E_{c} entre 500 et 3000 MPa.

3. Plaque d'ostéosynthèse selon l'une des revendications 1 ou 2, **caractérisée en ce que** le matériau du rembourrage en matière plastique (8) et du corps annulaire (11) est un matériau biorésorbable qui se décompose seulement après plusieurs semaines.

4. Plaque d'ostéosynthèse selon la revendication 3, **caractérisée en ce que** le matériau du rembourrage en matière plastique (8) et du corps annulaire (11) est un polylactide qui se décompose en environ 30 semaines.

5. Plaque d'ostéosynthèse selon l'une des revendications 1 à 4, **caractérisée en ce que** le rembourrage en matière plastique (8) est en appui sous forme de croissant sur la tête de vis (2a).

6. Plaque d'ostéosynthèse selon la revendication 5, **caractérisée en ce que** la forme de croissant présente une épaisseur de croissant (14) la plus grande dans la direction de l'axe longitudinal de la plaque d'ostéosynthèse, et **en ce que** la plus grande épaisseur de croissant (14) correspond au moins à 8 % du diamètre (17) de la tête de vis (2a) en appui.

7. Plaque d'ostéosynthèse selon l'une des revendications 1 à 6, **caractérisée en ce que** les surfaces d'appui (15, 16), faisant saillie au-dessus de la face inférieure (6), de la tête de vis (2a) et du corps annulaire (11) sont plus de 100 % supérieures à la section transversale du diamètre nominal du pas de vis de la vis à os (2).

8. Plaque d'ostéosynthèse selon l'une des revendications 1 à 7, **caractérisée en ce que** les corps annulaires (11) ne peuvent être chacun mis en place que dans une position dans un perçage (19) de la plaque d'ostéosynthèse et **en ce qu'**ils présentent une liaison par encliquetage (20) vers la plaque d'ostéosynthèse.

9. Plaque d'ostéosynthèse selon l'une des revendications 1 à 8, **caractérisée en ce que** sur leur face inférieure (6, 16), la plaque d'ostéosynthèse et le corps annulaire (11) sont bombés de forme concave en direction transversale.

10. Plaque d'ostéosynthèse selon l'une des revendications 1 à 9, **caractérisée en ce que** les têtes (2a) des vis à os (2) sont totalement enfoncées dans la plaque d'ostéosynthèse.

11. Plaque d'ostéosynthèse selon l'une des revendications 1 à 10, **caractérisée en ce que** la section transversale de la plaque d'ostéosynthèse (1) est affaiblie par des évidements (9) dans la région entre les perçages (19) pour y réaliser des régions à plus faible raideur à la flexion.

12. Plaque d'ostéosynthèse selon l'une des revendications 1 à 11, **caractérisée en ce qu'**elle est en titane ou en un alliage de titane.
